# NEUE EUROPÄISCHE PATENTSCHRIFT

(11) **EP 3 923 900 B2**
(45) Veröffentlichungstag und Bekanntmachung des Hinweises auf die Entscheidung über den Einspruch: **10.06.2026**
(45) Hinweis auf die Patenterteilung: 27.12.2023
(21) Anmeldenummer: 20702621.2
(22) Anmeldetag: 29.01.2020
(51) Int. Cl.: A61K 8/37, A61K 8/40, A61K 8/34, A61Q 17/04

(54) **GERUCHSSTABILE ETHANOLISCHE ZUBEREITUNG MIT OCTOCRYLEN**
ODOR-STABLE ETHANOLIC PREPARATION CONTAINING OCTOCRYLENE
PRÉPARATION ÉTHANOLIQUE CONTENANT DE L'OCTOCRYLÈNE, AYANT UNE ODEUR STABLE

(30) Priorität: 13.02.2019 DE 102019201861
(43) Veröffentlichungstag der Anmeldung: 22.12.2021
(73) Patentinhaber: Beiersdorf AG, 22529 Hamburg (DE)
(72) Erfinder: SPROCK, Sarah, 22297 Hamburg (DE); KORTENHORN, Annika, 25436 Tornesch (DE)
(74) Vertreter: Beiersdorf AG
(86) Internationale Anmeldenummer: PCT/EP2020/052087
(87) Internationale Veröffentlichungsnummer: WO 2020/164905

(56) Entgegenhaltungen:
- EP-A1- 3 173 130
- DE-A1- 102009 048 557

## Beschreibung

Die vorliegende Erfindung betrifft eine kosmetische Zubereitung enthaltend 2-Ethylhexyl-2-cyano-3,3-diphenylacrylat (Octocrylen), Ethanol und Propylheptylcaprylat (INCI Propylheptyl Caprylate).

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Ein vielfach in Tagespflegeprodukten und Sonnenschutzmitteln eingesetzter UV-Filter ist die Verbindung 2-Ethylhexyl-2-cyano-3,3-diphenylacrylat (Octocrylen), die neben ihren UV-Filtereigenschaften im UV-B-Bereich vor allem dazu verwendet wird den UV-A-Filter 4-(tert.-Butyl)-4'-methoxydibenzoylmethan gegen photochemischen Abbau zu stabilisieren.

Nachteilig am Stande der Technik ist dabei der Umstand, dass Octocrylen in Gegenwart von Ethanol in der Regel dazu führt, dass bei den Zubereitungen im Verlauf ihrer Lagerung sowie bei der Anwendung auf der Haut nach einer gewissen Zeit ein "Fehlgeruch" auftritt. Der entstehende Geruch erinnert entfernt an die aus der Küche bekannte Maggi-Stammwürze oder auch an den Geruch von Liebstöckel. Ursache für diesen Fehlgeruch ist die Bildung von Sotolon (3-Hydroxy-4,5-dimethyl-5H-furan-2-on), welches die folgende Struktur aufweist:

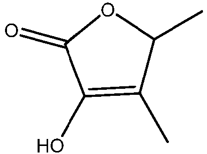

Der Reaktionsmechanismus, welcher zur Entstehung von 3-Hydroxy-4,5-dimethyl-5H-furan-2-on (im Folgenden Sotolon genannt) führt, ist bis heute unbekannt und unverstanden.

Eine Möglichkeit, den Fehlgeruch zu vermeiden, besteht natürlich darin, auf den Einsatz von Ethanol zu verzichten. Ethanol wird jedoch zunehmend dazu verwendet, die bei den Verbrauchern so beliebten "Konservierungsmittel freien" kosmetischen Zubereitungen vor mikrobiellem Befall zu schützen. Darüber hinaus bildet Ethanol aufgrund seiner Lösungseigenschaften, seiner sensorischen Eigenschaften sowie seines geringen Preises die ideale Grundlage für transparente Sprayzubereitungen, beispielsweise Sonnenschutzsprays. Nach dem Stand der Technik versucht man daher bei ethanolischen Zubereitungen, die geruchliche Fehlnote des Sotolons durch den Einsatz entsprechend hoher Mengen an Parfümstoffen zu maskieren. Diese Lösung hat jedoch den Nachteil, dass sich die Parfümstoffe im Laufe der Zeit aus der Zubereitung heraus verflüchtigen, während die Konzentration an Sotolon im Verlaufe der Zeit ansteigt. Darüber hinaus gibt es auch bei Kosmetika einen wachsenden Bedarf an sogenannten "parfümfreien" Zubereitungen, bei denen eine Geruchsmaskierung dann nicht möglich ist.

Es war daher die Aufgabe der vorliegenden Erfindung, eine kosmetische Zubereitung (insbesondere ein Tagespflegeprodukt oder ein Sonnenschutzmittel) enthaltend Octocrylen und Ethanol zu entwickeln, bei dem die Bildung eines Fehlgeruches und insbesondere die Bildung des Sotolons unterdrückt wird.

Erfindung ist definiert in den Ansprüchen.

Zwar kennt der Stand der Technik die DE102015214499.2, welche den Einsatz von Laurylalkoholdiphosphonsäure zur Geruchsstabilisierung offenbart, sowie die DE 102007017440.5, DE 102007017434.0, DE 102007017536.3, DE 102007017438.3, DE 102007017435.9, DE 102007024348.2, DE 102017213232.9 die den Einsatz von Propylheptylcaprylat in Kosmetika offenbaren, doch konnten diese Schriften nicht den Weg zur vorliegenden Erfindung weisen. Ferner kennt der Stand der Technik die EP3173130 und DE102009048557, die ebenfalls nicht den Weg zur vorliegenden Erfindung weisen konnten.

Es ist erfindungsgemäß vorteilhaft, wenn die Zubereitung 2-Ethylhexyl-2-cyano-3,3-diphenyl-acrylat (Octocrylen) in einer Gesamtmenge von 0,1 bis 10 Gewichts-%, bezogen auf das Gesamtgewicht der Zubereitung, enthält. Dabei ist ein Gehalt von 3 bis 9 Gewichts-%, bezogen auf das Gesamtgewicht der Zubereitung, erfindungsgemäß bevorzugt.

Ferner ist es erfindungsgemäß von Vorteil, wenn die Zubereitung Ethanol in einer Gesamtmenge von 0,1 bis 65 Gewichts-%, bezogen auf das Gesamtgewicht der Zubereitung, enthält. Dabei ist ein Ethanol-Gehalt von 3 bis 60 Gewichts-%, bezogen auf das Gesamtgewicht der Zubereitung, erfindungsgemäß bevorzugt.

Vorteilhafte Ausführungsformen der vorliegenden Erfindung sind dadurch gekennzeichnet, dass die Zubereitung Propylheptylcaprylat in einer Konzentration von 0,1 bis 25 Gewichts-%, bezogen auf das Gesamtgewicht der Zubereitung, beträgt. Hier beträgt die erfindungsgemäß bevorzugte Einsatzkonzentration von 0,5 bis 10 Gewichts-%, bezogen auf das Gesamtgewicht der Zubereitung. Propylheptylacprylat (IUPAC: 2-Propylheptyloctanoat) kann beispielsweise bei der BASF unter dem Handelsnamen Cetiol^{®} Sensoft erworben werden.

Es ist dabei erfindungsgemäß bevorzugt, wenn das Gewichtsverhältnis von Octocrylen zu Propylheptylcaprylat von 1:250 bis 1:0,01 beträgt.

Die erfindungsgemäße Zubereitung liegt als klare (transparente) ethanolische Lipidlösung vor.

Diese Ausführungsform der vorliegenden Erfindung sind dadurch gekennzeichnet, dass die Zubereitung transparent ist.

Dabei gilt eine Zubereitung erfindungsgemäß als transparent, wenn es möglich ist bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen (Schrifttyp Arial Schriftgröße 10), die sich unmittelbar hinter der Einmal-Küvette befinden, sollten erkennbar und lesbar sein.

Es ist erfindungsgemäß vorteilhaft, wenn die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornyliden-methyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxa-nyl]propyl]-phenol; 3-Benzylidencampher; Ethylhexylsalicylat; Terephthalidendicamphersulfonsäure und/oder deren Salze; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxy-4'-methylbenzophenon; 2,2'-Dihydroxy-4-methoxybenzophenon; 4-(tert.-Butyl)-4'-methoxydi-benzoylmethan; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phenoxy)propenyl)-meth-oxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhe-xylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Erfindungsgemäß bevorzugte Ausführungsformen der vorliegenden Erfindung sind dadurch gekennzeichnet, dass die Zubereitung Homomenthylsalicylat (Homosalate) und/oder Ethylhexylsalicylat enthält, wobei der Einsatz von Ethylhexylsalicylat erfindungsgemäß besonders bevorzugt ist.

Enthält die erfindungsgemäße Zubereitung Homomenthylsalicylat (Homosalate), so ist es erfindungsgemäß von Vorteil, diesen Stoff in einer Konzentration von 0,1 bis 15 Gewichts-%, bezogen auf das Gesamtgewicht der Zubereitung einzusetzen.

Enthält die erfindungsgemäße Zubereitung Ethylhexylsalicylat, so ist es erfindungsgemäß von Vorteil, diesen Stoff in einer Konzentration von 0,1 bis 5 Gewichts-%, bezogen auf das Gesamtgewicht der Zubereitung einzusetzen.

Enthält die erfindungsgemäße Zubereitung 4-(tert.-Butyl)-4'-methoxydibenzoylmethan, so ist es erfindungsgemäß von Vorteil, diesen Stoff in einer Konzentration von 0,1 bis 5 Gewichts-%, bezogen auf das Gesamtgewicht der Zubereitung einzusetzen.

Erfindungsgemäß vorteilhafte Ausführungsformen der vorliegenden Erfindung sind auch dadurch gekennzeichnet, dass die Zubereitung C12-15 Alkylbenzoat, Octyldodecanol, hydriertes Pflanzenöl (INCI: hydrogenated Vegetable Oil) und/oder Capryl/Caprinsäure Triglyceride enthält. Dabei ist insbesondere der Einsatz von hydriertes Pflanzenöl (INCI: hydrogenated Vegetable Oil) erfindungsgemäß bevorzugt.

Weiterhin ist es erfindungsgemäß vorteilhaft, wenn die Zubereitung ein oder mehrere Komplexbildner aus der Gruppe der Verbindungen
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze und/ oder deren Amin-N-Oxide enthält.

Es ist erfindungsgemäß von Vorteil, wenn die Zubereitung ein oder mehrere Polysaccharide ausgewählt aus der Gruppe der Gumen enthält. Dabei ist es insbesondere bevorzugt, die Verbindungen Welan Gum, Sclerotium Gum und/oder Cellulose Gum oder Verbindungen aus der Gruppe der Alginate und Carboxymethylcellulose einzusetzen. Erfindungsgemäß besonders bevorzugt ist dabei der Einsatz von Cellulose Gum und Carboxymethylcellulose.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Es gibt darüber hinaus erfindungsgemäß vorteilhafte Ausführungsformen, die dadurch gekennzeichnet sind, dass die Zubereitung Polyvinylalkohol und/oder Triacontanyl PVP und/oder Acrylates/Octylacrylamide Copolymer enthält.

Weiterhin ist es erfindungsgemäß von Vorteil, wenn die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Polidocanol, Glycerylglycose, (2-Hydroxyethyl)harnstoff, Vitamin E bzw. seine Derivate, Hyaluronsäure und/oder deren Salze und/oder Licochalcon A enthält.

Es ist vorteilhaft im Sinne der vorliegenden Erfindung, wenn die Zubereitung ein oder mehrere Verdickungsmittel enthält.

Erfindungsgemäß bevorzugt werden dabei ein oder mehrere Verdickungsmittel gewählt aus der Gruppe der Verbindungen Ammonium Acryloyldimethyltaurate/VP Copolymer, Ammonium Acryloyldimethyltaurate/ Beheneth-25 Methacrylate Copolymer, Hydroxypropylcellulose Vinylpyrrolidon/Acrylsäure-Copolymer enthält.

Es ist insbesondere von Vorteil, die Filmbildner aus der Gruppe der Polymere auf Basis von Polyvinylpyrrolidon (PVP) 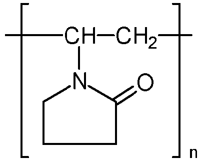 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß besonders bevorzugt ist der Einsatz von Acrylat/Octylacralymid Copolymer (Dermacryl 79).

Erfindungsgemäß ist auch ein Verfahren zur Geruchsstabilisierung von ethanolhaltigen, kosmetischen Zubereitungen enthaltend 2-Ethylhexyl-2-cyano-3,3-diphenylacrylat (Octocrylen), dadurch gekennzeichnet, dass man der Zubereitung Propylheptylcaprylat (INCI: Propylheptyl Caprylate) zusetzt sowie die Verwendung von Propylheptylcaprylat (INCI: Propylheptyl Caprylate) zur Geruchsstabilisierung von 2-Ethylhexyl-2-cyano-3,3-diphenylacrylat (Octocrylen) enthaltenden ethanolischen kosmetischen Zubereitungen.

Die erfindungsgemäße Zubereitung kann vorteilhaft als Creme, Lotion, Gel, Aerosol oder Spray dargereicht werden.

### Vergleichsversuch

Es wurden die folgenden Rezepturen hergestellt und ihre Geruchsstabilität miteinander verglichen:

| **INCI** | **Muster 1** (Referenz) **(m%)** | **Muster 2** (Muster 1 enthaltend Propylheptyl Caprylate statt Butylene Glycol Dicaprylate/Dicaprate) **m(%)** |
|---|---|---|
| Dibutyl Adipate | 3 | 3 |
| C12-15 Alkyl Benzoate | 9,5 | 9,5 |
| Butylene Glycol Dicaprylate/Dicaprate | 3 | 0 |
| Propylheptyl Caprylate | 0 | 3 |
| Acrylates/Octylacrylamide Copolymer | 1 | 1 |
| Parfum | 0,7 | 0,7 |
| Glycerin | 2,5 | 2,5 |
| Alcohol Denat. | 50,3 | 50,3 |
| Homosalate | 9 | 9 |
| Octocrylene | 9 | 9 |
| Ethylhexyl Salicylate | 4,5 | 4,5 |
| Bis-Ethylhexyloxyphenol Methoxyphenyl Triazine | 3 | 3 |
| Butyl Methoxydibenzoylmethane | 4,5 | 4,5 |

Die Muster wurden in Glas-Vials unter folgenden Bedingungen bestrahlt:

| | |
|---|---|
| Gerät: | Atlas Suntester |
| Filterkombination: | Beschichtetes Quarzglas 56052388, und UV 56052371 |
| Bestrahlungsstärke: | 765 W/m2 |
| Bestrahlungszeit: | 72 h |

Anschließend wurde der Geruch der Muster von 5 Experten beurteilt: Alle 5 Experten nehmen bei Muster 1 einen deutlich ausgeprägteren Fehlgeruch als bei Muster 2 wahr.

### Beispiele

Die nachfolgenden Beispiele sollen die vorliegende Erfindung verdeutlichen. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Bsp.1: Transparentes Sonnenschutzspray SPF 20

| **INCI** | **m(%)** |
|---|---|
| Propylheptyl Caprylate | 2,00 |
| Isopropyl Palmitate | 3,00 |
| C12-15 Alkyl Benzoate | 10,00 |
| Acrylates/Octylacrylamide Copolymer | 1,00 |
| Parfum | 0,70 |
| Glycerin | 4,00 |
| Alcohol Denat. | 56,30 |
| Homosalate | 9,00 |
| Octocrylene | 5,00 |
| Ethylhexyl Salicylate | 4,50 |
| Butyl Methoxydibenzoylmethane | 4,50 |

### Bsp. 2: Transparentes Sonnenschutzspray SPF 50

| **INCI** | **m(%)** |
|---|---|
| Dibutyl Adipate | 3,00 |
| Propylheptyl Caprylate | 3,00 |
| C12-15 Alkyl Benzoate | 9,00 |
| Acrylates/Octylacrylamide Copolymer | 1,80 |
| Parfum | 0,70 |
| Glycerin | 2,50 |
| Alcohol Denat. | 49,40 |
| Diethylamino Hydroxybenzoyl Hexyl Benzoate | 0,90 |
| Homosalate | 9,00 |
| Octocrylene | 9,00 |
| Ethylhexyl Salicylate | 4,50 |
| Bis-Ethylhexyloxyphenol Methoxyphenyl Triazine | 1,70 |
| Ethylhexyl Triazone | 1,00 |
| Butyl Methoxydibenzoylmethane | 4,50 |

### Bsp. 3 Sonnenschutzgel SPF50

| **INCI** | **m(%)** |
|---|---|
| Cocoglycerides | 0,50 |
| C12-15 Alkyl Benzoate | 5,50 |
| Acrylates/Octylacrylamide Copolymer | 2,50 |
| Parfum | 0,80 |
| Glycerin | 3,00 |
| Propylheptyl Caprylate | 2,00 |
| Cetyl Alcohol | 5,00 |
| Hydroxypropylcellulose | 0,90 |
| Alcohol Denat. | 47,55 |
| Homosalate | 9,50 |
| Octocrylene | 9,00 |
| Ethylhexyl Salicylate | 4,50 |
| Bis-Ethylhexyloxyphenol Methoxyphenyl Triazine | 2,25 |
| Ethylhexyl Triazone | 2,25 |
| Butyl Methoxydibenzoylmethane | 4,75 |

## Patentansprüche

1. Verwendung von Propylheptylcaprylat (INCI: Propylheptyl Caprylate) in einer kosmetischen Zubereitung enthaltend
a) 2-Ethylhexyl-2-cyano-3,3-diphenylacrylat (Octocrylen) und
b) Ethanol,
wobei die Zubereitung weniger als 1 Gewichts-% Wasser, bezogen auf das Gesamtgewicht der Zubereitung, enthält, zur Unterdrückung der Bildung eines Fehlgeruches, insbesondere der Bildung von Sotolon.

2. Verwendung nach Anspruch 1, **dadurch gekennzeichnet, dass** die Zubereitung 2-Ethylhexyl-2-cyano-3,3-diphenylacrylat (Octocrylen) in einer Gesamtmenge von 0,1 bis 10 Gewichts-%, bezogen auf das Gesamtgewicht der Zubereitung, enthält.

3. Verwendung nach einem der vorhergehenden Ansprüche, **dadurch gekennzeichnet, dass** die Zubereitung Ethanol in einer Gesamtmenge von 0,1 bis 65 Gewichts-%, bezogen auf das Gesamtgewicht der Zubereitung, enthält.

4. Verwendung nach einem der vorhergehenden Ansprüche, **dadurch gekennzeichnet, dass** die Zubereitung Propylheptylcaprylat in einer Konzentration von 0,1 bis 25 Gewichts-%, bezogen auf das Gesamtgewicht der Zubereitung, beträgt.

5. Verwendung nach einem der vorhergehenden Ansprüche, **dadurch gekennzeichnet, dass** das Gewichtsverhältnis von Octocrylen zu Propylheptylcaprylat von 1:250 bis 1:0,01 beträgt.

6. Verwendung nach einem der vorhergehenden Ansprüche, **dadurch gekennzeichnet, dass** die Zubereitung transparent ist.

7. Verwendung nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Ethylhexylsalicylat; Terephthalidendicamphersulfonsäure und/oder deren Salze; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

8. Verwendung nach einem der vorhergehenden Ansprüche, **dadurch gekennzeichnet, dass** die Zubereitung Homomenthylsalicylat (Homosalate) und/oder Ethylhexylsalicylat enthält.

9. Verwendung nach einem der vorhergehenden Ansprüche, **dadurch gekennzeichnet, dass** die Zubereitung frei ist von Propylparaben, Butylparaben, Methylisothiazolinon, Chlormethylisothiazolinon, IPBC, DMDM-Hydantoin, Dimethylol Glycol, Dimethylol Urea, Sodium Hydroxmethyl Glycinate, BHT, 3-(4-Methylbenzyliden)campher, 2-Hydroxy-4-methoxybenzophenon (INCI: Oxybenzon), 4-Methoxyzimtsäureisoamylester, 4-Methoxyzimtsäureethylhexylestern und Cyclomethicon.

10. Verwendung nach einem der vorhergehenden Ansprüche, **dadurch gekennzeichnet, dass** die Zubereitung C12-15 Alkylbenzoat, Octyldodecanol, hydriertes Pflanzenöl (INCI: hydrogenated Vegetable Oil) und/oder Capryl/Caprinsäure Triglyceride enthält.

11. Verwendung nach einem der vorhergehenden Ansprüche, **dadurch gekennzeichnet, dass** die Zubereitung ein oder mehrere Komplexbildner aus der Gruppe der Verbindungen
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze und/ oder deren Amin-N-Oxide enthält.

12. Verwendung nach einem der vorhergehenden Ansprüche, **dadurch gekennzeichnet, dass** die Zubereitung Polysaccharide ausgewählt werden aus der Gruppe der Gumen, insbesondere Welan Gum, Sclerotium Gum und/oder Cellulose Gum oder aus der Gruppe der Verbindungen Alginate und Carboxymethylcellulose, enthält.

13. Verwendung nach einem der vorhergehenden Ansprüche, **dadurch gekennzeichnet, dass** die Zubereitung Polyvinylalkohol und/oder Triacontanyl PVP enthält.

14. Verwendung nach einem der vorhergehenden Ansprüche, **dadurch gekennzeichnet, dass** die Zubereitung Acrylat/Octylacralymid Copolymer enthält.

15. Verwendung nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Polidocanol, Glycerylglycose, (2-Hydroxyethyl)harnstoff, Vitamin E bzw. seine Derivate, Hyaluronsäure und/oder deren Salze und/oder Licochalcon A enthält.

## Claims

1. Use of propylheptyl caprylate (INCI: Propylheptyl Caprylate) in a cosmetic preparation comprising
a) 2-ethylhexyl 2-cyano-3,3-diphenylacrylate (octocrylene) and
b) ethanol,
wherein the preparation comprises less than 1% by weight of water, based on the total weight of the preparation, to suppress the formation of an unpleasant odour, in particular the formation of sotolon.

2. Use according to Claim 1, **characterized in that** the preparation comprises 2-ethylhexyl 2-cyano-3,3-diphenylacrylate (octocrylene) in a total amount of 0.1% to 10% by weight, based on the total weight of the preparation.

3. Use according to either of the preceding claims, **characterized in that** the preparation comprises ethanol in a total amount of 0.1% to 65% by weight, based on the total weight of the preparation.

4. Use according to any of the preceding claims, **characterized in that** the preparation comprises propylheptyl caprylate at a concentration of 0.1% to 25% by weight, based on the total weight of the preparation.

5. Use according to any of the preceding claims, **characterized in that** the ratio by weight of octocrylene to propylheptyl caprylate is from 1:250 to 1:0.01.

6. Use according to any of the preceding claims, **characterized in that** the preparation is transparent.

7. Use according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-benzylidenecamphor; ethylhexyl salicylate; terephthalidenedicamphorsulfonic acid and/or salts thereof; 2-ethylhexyl 4-(dimethylamino)benzoate; amyl 4-(dimethylamino)benzoate; di(2-ethylhexyl) 4-methoxybenzalmalonate; 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate (homosalate); 2-ethylhexyl 2-hydroxybenzoate; dimethicodiethylbenzalmalonate; 3-(4-(2,2-bisethoxycarbonylvinyl)phenoxy)propenyl)methoxysiloxane / dimethylsiloxane copolymer; 4-(tert-butyl)-4'-methoxydibenzoylmethane; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanines; titanium dioxide; zinc oxide.

8. Use according to any of the preceding claims, **characterized in that** the preparation comprises homomenthyl salicylate (homosalate) and/or ethylhexyl salicylate.

9. Use according to any of the preceding claims, **characterized in that** the preparation is free from propylparaben, butylparaben, methylisothiazolinone, chloromethylisothiazolinone, IPBC, DMDM hydantoin, dimethylol glycol, dimethylolurea, sodium hydroxymethylglycinate, BHT, 3-(4-methylbenzylidene)camphor, 2-hydroxy-4-methoxybenzophenone (INCI: Oxybenzone), isoamyl 4-methoxycinnamate, ethylhexyl 4-methoxycinnamate and cyclomethicone.

10. Use according to any of the preceding claims, **characterized in that** the preparation comprises C12-15 alkyl benzoate, octyldodecanol, hydrogenated vegetable oil (INCI: Hydrogenated Vegetable Oil) and/or caprylic/capric acid triglycerides.

11. Use according to any of the preceding claims, **characterized in that** the preparation comprises one or more chelating agents from the group of compounds comprising
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- iminodisuccinate
- sodium polyphosphate
- tetrasodium pyrophosphate
- hydroxamic acid
- polygalacturonic acid
- succinic acid
- formic acid
- malic acid
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- iminodisuccinate
- sodium polyphosphate
- tetrasodium pyrophosphate
- hydroxamic acid
- polygalacturonic acid
- succinic acid
- formic acid
- malic acid
- ethylendiaminetetraacetic acid (EDTA)
and/or alkali metal salts and/or amine N-oxides thereof.

12. Use according to any of the preceding claims, **characterized in that** the preparation comprises polysaccharides selected from the group of gums, in particular welan gum, sclerotium gum and/or cellulose gum or from the group of compounds comprising alginates and carboxymethyl cellulose.

13. Use according to any of the preceding claims, **characterized in that** the preparation comprises polyvinyl alcohol and/or Triacontanyl PVP.

14. Use according to any of the preceding claims, **characterized in that** the preparation comprises acrylate/octylacrylamide copolymer.

15. Use according to any of the preceding claims, **characterized in that** the preparation comprises one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopheryl acetate, dihydroxyacetone, 8-hexadecene-1,16-dicarboxylic acid, polidocanol, glycerylglucose, (2-hydroxyethyl)urea, vitamin E and derivatives thereof, hyaluronic acid and/or salts thereof, and/or licochalcone A.

## Revendications

1. Utilisation de caprylate de propylheptyle (INCI : Propylheptyl Caprylate) dans une préparation cosmétique, contenant
a) du 2-cyano-3,3-diphénylacrylate de 2-éthylhexyle (octocrylène) et
b) de l'éthanol,
la préparation contenant moins de 1 % en poids d'eau par rapport au poids total de la préparation, pour l'inhibition de la formation d'une mauvaise odeur, en particulier de la formation de sotolon.

2. Utilisation selon la revendication 1, **caractérisée en ce que** la préparation contient du 2-cyano-3,3-diphénylacrylate de 2-éthylhexyle (octocrylène) en une quantité totale de 0,1 à 10 % en poids par rapport au poids total de la préparation.

3. Utilisation selon l'une des revendications précédentes, **caractérisée en ce que** la préparation contient de l'éthanol en une quantité totale de 0,1 à 65 % en poids par rapport au poids total de la préparation.

4. Utilisation selon l'une des revendications précédentes, **caractérisée en ce que** la préparation contient du caprylate de propylheptyle à une concentration de 0,1 à 25 % en poids par rapport au poids total de la préparation.

5. Utilisation selon l'une des revendications précédentes, **caractérisée en ce que** le rapport en poids de l'octocrylène au caprylate de propylheptyle est de 1:250 à 1:0,01.

6. Utilisation selon l'une des revendications précédentes, **caractérisée en ce que** la préparation est transparente.

7. Utilisation selon l'une des revendications précédentes, **caractérisée en ce que** la préparation contient un ou plusieurs filtres UV choisis dans le groupe des composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-benzylidènecamphre ; salicylate d'éthylhexyle ; acide téréphtalidènedicamphosulfonique et/ou ses sels ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle (homosalate) ; 2-hydroxybenzoate de 2-éthylhexyle ; malonate de diméthicodiéthylbenzal ; copolymère 3-(4-(2,2-bis-éthoxycarbonylvinyl)-phénoxy)propényl)-méthoxysiloxane / diméthylsiloxane ; 4-(tert-butyl)-4'-méthoxydibenzoylméthane ; ester hexylique de l'acide 2-(4'-diéthylamino-2'-hydroxybenzoyl)-benzoïque ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine portant le numéro CAS 288254-16-0 ; 2,4-bis-{[4-(2-éthyl-hexyloxy)-2-hydroxy]-phényl}-6-(4-méthoxyphényl)-1,3,5-triazine (INCI : Bis--Ethylhexyloxyphenol Methoxyphenyl Triazin) ; ester tris(2-éthylhexylique de l'acide 4,4',4"-(1,3,5-triazine-2,4,6-triyltriimino)-tris-benzoïque (aussi : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc.

8. Utilisation selon l'une des revendications précédentes, **caractérisée en ce que** la préparation contient du salicylate d'homomenthyle (homosalate) et/ou du salicylate d'éthylhexyle.

9. Utilisation selon l'une des revendications précédentes, **caractérisée en ce que** la préparation est exempte de propylparabène, de butylparabène, de méthylisothiazolinone, de chlorométhylisothiazolinone, d'IPBC, de DMDM-hydantoïne, de diméthylolglycol, de diméthylolurée, d'hydroxyméthylglycinate de sodium, de BHT, de 3-(4-méthylbenzylidène)camphre, de 2-hydroxy-4-méthoxybenzophénone (INCI : Oxybenzon), de l'ester isoamylique de l'acide 4-méthoxycinamique, des esters éthylhexylique de l'acide 4-méthoxycinamique et de cyclométhicone.

10. Utilisation selon l'une des revendications précédentes, **caractérisée en ce que** la préparation contient un benzoate d'alkyle en C12-15, de l'octyldodécanol, une huile végétale hydrogénée (INCI : hydrogenated Vegetable Oil) et/ou des triglycérides de l'acide caprylique/caprique.

11. Utilisation selon l'une des revendications précédentes, **caractérisée en ce que** la préparation contient un ou plusieurs complexants du groupe des composés
- acide 1-hydroxyéthane-(1,1-diphosphonique)/HEDP
- acide aminotriméthylènephosphonique/ATMP
- acide diéthylènetriaminepenta(méthylènephosphonique)/ DTPMP
- acide éthylènediaminetétra(méthylènephosphonique)/ EDTMP
- acide phosphonobutane-tricarboxylique/PBTC
- iminodisuccinate
- polyphosphate de sodium
- pyrophosphate tétrasodique
- acide hydroxamique
- acide polygalacturonique
- acide succinique
- acide formique
- acide malique
- acide 1-hydroxyéthane-(1,1-diphosphonique)/HEDP
- acide aminotriméthylènephosphonique/ATMP
- acide diéthylènetriaminepenta(méthylènephosphonique)/ DTPMP
- acide éthylènediaminetétra(méthylènephosphonique)/ EDTMP
- acide phosphonobutane-tricarboxylique/PBTC
- iminodisuccinate
- polyphosphate de sodium
- pyrophosphate tétrasodique
- acide hydroxamique
- acide polygalacturonique
- acide succinique
- acide formique
- acide malique
- acide éthylènediaminetétraacétique (EDTA)
et/ou leurs sels de métaux alcalins et/ou leurs N-oxydes d'amine.

12. Utilisation selon l'une des revendications précédentes, **caractérisée en ce que** la préparation contient des polysaccharides choisis dans le groupe des gommes, en particulier la gomme Welan, la gomme Sclerotium et/ou la gomme de cellulose, ou dans le groupe des composés alginates et carboxyméthylcellulose.

13. Utilisation selon l'une des revendications précédentes, **caractérisée en ce que** la préparation contient du poly(alcool vinylique) et/ou du triacontanyle PVP.

14. Utilisation selon l'une des revendications précédentes, **caractérisée en ce que** la préparation contient un copolymère acrylate/octylacrylamide.

15. Utilisation selon l'une des revendications précédentes, **caractérisée en ce que** la préparation contient un ou plusieurs composés choisis dans le groupe des composés acide alpha-lipoïque, acide folique, phytoène, D-biotine, Coenzyme Q10, alpha-glucosylrutine, carnitine, carnosine, isoflavonoïdes naturels et/ou synthétiques, flavonoïdes, créatine, créatinine, taurine, β-alanine, panthénol, magnolol, honokiol, acétate de tocophéryle, dihydroxyacétone ; acide 8-hexadécène-1,16-dicarboxylique, polidocanol, glycérylglycose, (2-hydroxyéthyl)urée, vitamine E ou ses dérivés, acide hyaluronique et/ou ses sels et/ou licochalcone A.
